# DEMANDE DE BREVET EUROPEEN

(11) **EP 1 364 629 A1**
(43) Date de publication de la demande: **26.11.2003**
(21) Numéro de dépôt: 03291187.7
(22) Date de dépôt: 20.05.2003
(51) Int. Cl.: A61F 2/06

(54) **Prothèse à collerette à couture radiale et périphérique**

(30) Priorité: 24.05.2002 FR 0206387
(71) Demandeur: Laboratoires Perouse, 60540 Bornel (FR)
(72) Inventeur: Perouse, Eric, 75016 Paris (FR)
(74) Mandataire: Blot, Philippe Robert Emile

(57) **Abrégé**

La prothèse à collerette comporte un corps tubulaire muni à une extrémité d'une collerette extérieure adaptée pour l'anastomose de la prothèse sur un conduit tubulaire muni d'une ouverture, dans laquelle ladite collerette est confectionnée dans un matériau textile et comporte au moins une couture (324, 426) assurant sa mise en forme à l'extrémité du corps tubulaire. Ladite collerette comporte une première couronne solidaire, suivant son contour intérieur, de l'extrémité du corps tubulaire et une seconde couronne (24) entourant ledit corps tubulaire et s'étendant sensiblement contre la première couronne (24) en étant reliées l'une à l'autre suivant leurs contours extérieurs. La première couronne est venue de matière avec le corps tubulaire, et les première et seconde couronnes sont liées entre elles par une couture périphérique extérieure (426).

## Description

La présente invention concerne une prothèse à collerette, du type comportant un corps tubulaire muni à une extrémité d'une collerette extérieure adaptée pour l'anastomose de la prothèse sur un conduit tubulaire muni d'une ouverture, dans laquelle ladite collerette est confectionnée dans un matériau textile et comporte au moins une couture assurant sa mise en forme à l'extrémité du corps tubulaire, ladite collerette comportant une première couronne solidaire, suivant son contour intérieur, de l'extrémité du corps tubulaire et une seconde couronne entourant ledit corps tubulaire et s'étendant sensiblement contre la première couronne en étant reliées l'une à l'autre suivant leurs contours extérieurs.

Elle concerne en outre un procédé de fabrication d'une telle prothèse à collerette.

Les prothèses à collerette de ce type sont utilisées en tant que prothèses vasculaires, en chirurgie vasculaire, pour traiter les vaisseaux sanguins malades, notamment des portions d'artères, par pontage ou par substitution.

Comme illustré dans le document FR-A-2.751.867 décrivant une telle prothèse, ces prothèses sont reliées à un conduit organique tubulaire tel qu'une veine ou une artère, en regard d'une ouverture ménagée sur ce conduit.

La collerette, qui est prévue à l'extrémité du corps tubulaire permet la liaison de la prothèse à l'aide de clips ou d'agrafes déformables plastiquement ou élastiquement.

La prothèse à collerette décrite dans le document FR-A-2.751.867 est réalisée soit par thermoformage de son tronçon d'extrémité, ou encore par collage ou soudage d'une collerette séparée rapportée à l'extrémité du corps tubulaire.

Dans ce document, seules les technologies pouvant être mises en oeuvre sont abordées, sans que les procédés de fabrication proprement dits de cette prothèse à collerette ne soient décrits précisément.

Ainsi, il est très malaisé avec ces technologies de réaliser simplement une prothèse à collerette, dont la structure est satisfaisante et qui puisse être implantée de manière fiable dans le corps humain.

D'autres solutions sont proposées dans la demande de brevet 2.799.362.

L'invention a pour but de proposer une prothèse à collerette pouvant être fabriquée industriellement et ayant une fiabilité satisfaisante pour être implantée durablement dans le corps humain, ainsi que son procédé de fabrication.

A cet effet, l'invention a pour objet une prothèse à collerette du type précité, caractérisée en ce que la première couronne est venue de matière avec le corps tubulaire, et les première et seconde couronnes sont liées entre elles par une couture périphérique extérieure.

Suivant des modes particuliers de réalisation, la prothèse comporte l'une ou plusieurs des caractéristiques suivantes :
- la première couronne comporte au moins une couture transversale ;
- la ou chaque couture transversale s'étend sensiblement radialement; et
- la première couronne comporte une unique couture transversale .

L'invention a en outre pour objet un procédé de fabrication d'une prothèse à collerette, du type comportant un corps tubulaire muni à une extrémité d'une collerette extérieure adaptée pour l'anastomose de la prothèse sur un conduit tubulaire muni d'une ouverture, caractérisé en ce qu'il comporte les étapes suivantes :
- fabriquer une ébauche de prothèse en matière textile comportant une jupe tubulaire à une extrémité de laquelle débouche au moins un bras tubulaire destiné à former le corps tubulaire de la prothèse, le diamètre de la jupe tubulaire étant supérieur au diamètre du bras,
- découper la jupe tubulaire autour et à l'écart du bras tubulaire pour former une première couronne prolongeant le corps tubulaire,
- fabriquer dans un matériau textile un élément annulaire sensiblement plat, ledit élément ayant un contour extérieur sensiblement identique à celui de la première couronne ; et
- coudre ensemble ledit élément annulaire à ladite première couronne par une couture périphérique ménagée suivant leurs contours extérieurs.

Suivant des modes particuliers de mise en oeuvre, le procédé comporte l'un ou plusieurs des caractéristiques suivantes :
- il comporte pour la réalisation de la première couronne une étape de mise en plis de la jupe tubulaire autour d'un mannequin rigide comportant au moins une partie en forme de disque autour de laquelle est confectionnée la collerette ; et
- l'étape de fabrication de l'ébauche comporte :
   . une étape de fabrication d'une ébauche primaire comportant une jupe tubulaire à une extrémité de laquelle débouche un premier et un second bras tubulaires, le premier bras est destiné à former le corps tubulaire de la prothèse ;
   . une étape de découpe du second bras tubulaire au niveau de la jupe tubulaire ;
   . une étape d'obturation par couture de l'orifice obtenu par la découpe du second bras.

L'invention sera mieux comprise à la lecture de la description qui va suivre, donnée uniquement à titre d'exemple et faite en se référant aux dessins, sur lesquels :
- la figure 1 est une vue en coupe longitudinale d'une prothèse à collerette en cours d'implantation ;
- la figure 2 est une vue en perspective d'un outil pour la fabrication d'une prothèse à collerette selon l'invention ;
- la figure 3 est une vue en perspective d'une ébauche de prothèse vasculaire utilisée pour la fabrication de la prothèse à collerette ;
- les figures 4 et 5 sont des vues de côté en perspective illustrant deux étapes successives du procédé de fabrication de la prothèse ;
- les figures 6 et 7 sont des vues en coupe de côté illustrant les étapes suivantes du procédé de fabrication de la prothèse ;
- la figure 8 est une vue en perspective de la prothèse à collerette selon l'invention en cours de fabrication ; et
- la figure 9 est une vue en perspective et en coupe de la prothèse à collerette assemblée.

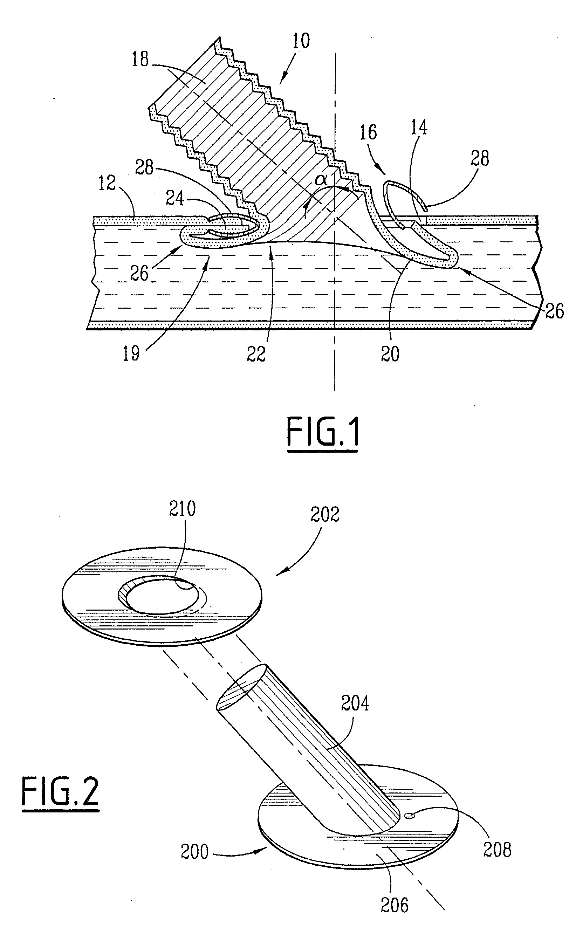

Sur la figure 1 est représentée généralement une prothèse à collerette 10 en cours d'implantation sur un conduit tubulaire 12 tel qu'une artère. Ce conduit est muni, dans sa paroi latérale, d'une ouverture 14, à la périphérie de laquelle la prothèse 10 est reliée grâce à des moyens de fixation 16.

La prothèse 10 comporte un corps tubulaire souple 18 et une collerette d'extrémité 19.

Le corps 18 est constitué d'un conduit tubulaire annelé, c'est-à-dire présentant sur l'essentiel de sa longueur une déformation permanente en hélice. Ce type de déformation est également connu sous le nom de "cosselage".

A son extrémité de raccordement portant la collerette 19, le corps tubulaire 18 est prolongé par une première couronne 20. L'axe de la première couronne 20 définit avec l'axe du corps tubulaire 18 un angle α sensiblement égal à 45°.

Le corps 18 et la première couronne 20 sont reliés l'un à l'autre suivant le contour intérieur, noté 22, de la première couronne.

Une seconde couronne 24 prolonge la première couronne 22. Celle-ci est sensiblement appliquée sur la face de la première couronne 20 prolongeant la surface extérieure du corps 18. Elle entoure ainsi l'extrémité du corps 18.

Les première et seconde couronnes sont reliées suivant leur contour extérieur 26 et forment ensemble la collerette 19.

Comme illustré sur la figure 1, les moyens 16 de liaison de la prothèse à collerette avec le conduit tubulaire 12 comportent un ensemble de clips 28 engagés au travers de l'ouverture 14 en prenant appui d'une part sur la surface extérieure du conduit tubulaire 12 et d'autre part sur la seconde couronne 24, assurant ainsi le plaquage de cette seconde couronne contre la surface intérieure du conduit tubulaire 12.

Sur la figure 2 est représenté un outil de fabrication d'une prothèse à collerette selon l'invention.

Cet outil comporte un corps d'outil 200 et une rondelle 202. Ces deux éléments sont formés en métal.

Le corps d'outil comporte une tige rectiligne 204 à une extrémité de laquelle est fixé un disque elliptique 206. L'extrémité de la tige 204 recevant le disque est biseauté à 45°, de sorte que l'axe du disque 206 définit avec l'axe de la tige 204 un angle voisin de 45°. L'axe de la tige 204 passe sensiblement par le centre du disque elliptique 206. L'axe de la tige 204 se projette orthogonalement sur le disque 206 suivant le grand axe du disque.

Un trou débouchant 208 traverse le disque 206. Ce trou est ménagé à la base de la tige rectiligne 204 suivant le grand axe de l'ellipse du côté où la tige 204 définit avec le disque 206 un angle maximal de 135°.

La rondelle 202 présente une forme générale elliptique. Son contour extérieur correspond au contour du disque elliptique 206.

La rondelle est traversée en son centre par une lumière elliptique 210 dont la section est légèrement supérieure à la section de la tige rectiligne 204 prise suivant un plan parallèle au disque 206. La paroi latérale de la lumière 210, définie dans l'épaisseur de la rondelle 202, est biseautée suivant une enveloppe cylindrique pour correspondre à l'inclinaison de la tige 204.

Ainsi, la tige 204 peut être introduite dans la lumière 210, assurant ainsi un coulissement de la rondelle 202 suivant la longueur de la tige 204 en étant maintenue parallèle au disque 206. Dans sa position extrême, la rondelle 202 s'applique exactement sur la surface dite supérieure du disque elliptique 206.

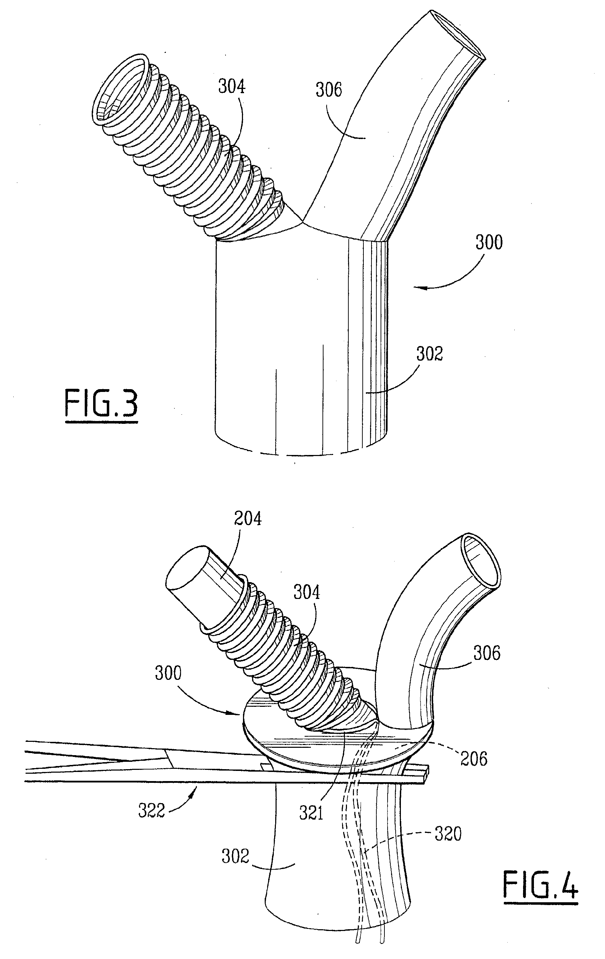

Afin de réaliser la prothèse, on utilise une ébauche primaire 300 représentée sur la figure 3. Celle-ci est constituée d'une prothèse vasculaire bifurquée. Cette prothèse est normalement utilisée pour assurer la liaison d'un tronçon principal à deux tronçons de dérivation. Elle a ainsi généralement la forme d'un Y.

Cette prothèse bifurquée est réalisée par tricotage suivant un procédé connu en soi. Elle comporte un corps principal défini par une jupe tubulaire 302 ayant un diamètre d'environ 3 cm. Le corps est prolongé par deux bras adjacents 304, 306. Ceux-ci sont formés par des conduits tubulaires débouchant côte à côte à une extrémité du corps. Chaque conduit tubulaire a un diamètre sensiblement égal à la moitié du diamètre de la jupe 302.

L'un des bras 304 est annelé par mise en oeuvre d'un procédé connu en soi. En revanche, la jupe 302 et le bras 306 sont lisses, ceux-ci n'ayant subi aucun traitement préalable de mise en forme.

Pour la fabrication de la prothèse, et comme illustré sur la figure 4, le corps d'outil 200 est d'abord introduit dans l'ébauche 300 depuis l'extrémité ouverte de la jupe délimitant le corps principal. Plus précisément, la tige 204 du corps d'outil est engagée au travers du bras 304, le disque elliptique 206 étant disposé dans la région de liaison de la jupe 302 aux bras 304 et 306. En particulier, l'extrémité du bras 306 est appliquée sur la face supérieure du disque elliptique 206 dans le plan comportant le trou 206, c'est-à-dire dans la région du disque délimitant avec l'axe de la tige 204 l'angle maximal.

La paroi cylindrique de la jupe 302 est appliquée sur la surface supérieure du disque elliptique 206. Afin d'assurer un plaquage satisfaisant de la jupe 302 sur la surface supérieure du disque 206, un fil 320 formant une boucle est engagé autour de la base du bras 304 porté par la tige 204, les deux brins du fil étant introduits dans le trou 206. Ces derniers traversent ainsi l'ébauche 300 dans la région de liaison des bras 304 et 306. Les deux extrémités du fil s'étendent alors à l'intérieur de la jupe 302 et débouchent à son extrémité ouverte. La traction sur les deux extrémités du fil assurent que la jupe 302 vienne en contact intime avec toute la surface supérieure du disque 206, créant ainsi une ligne de pliage périphérique 321 à l'extrémité du bras 304.

Afin d'assurer la retenue de l'ensemble, une pince 322 est mise en place transversalement sur toute la largeur de la jupe 302. Celle-ci est disposée au-dessous du disque 206, assurant ainsi une traction sur la partie de la jupe 302 entourant le disque 206.

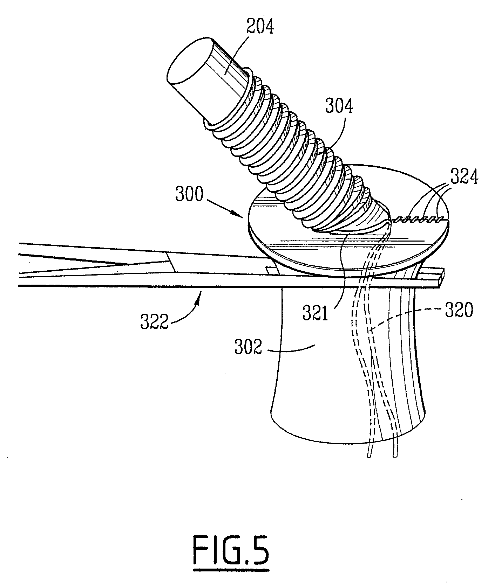

A l'étape suivante, le bras 306 est sectionné dans le plan du disque 206 et les deux bords ainsi définis à l'extrémité de la jupe 302 sont raboutés par une couture 324 réalisée manuellement, comme illustré sur la figure 5. La région annulaire ainsi délimitée dans la jupe 302 sur la face supérieure du disque elliptique 206 forme la première couronne 20 de la prothèse. La couture 324 s'étend radialement suivant cette couronne.

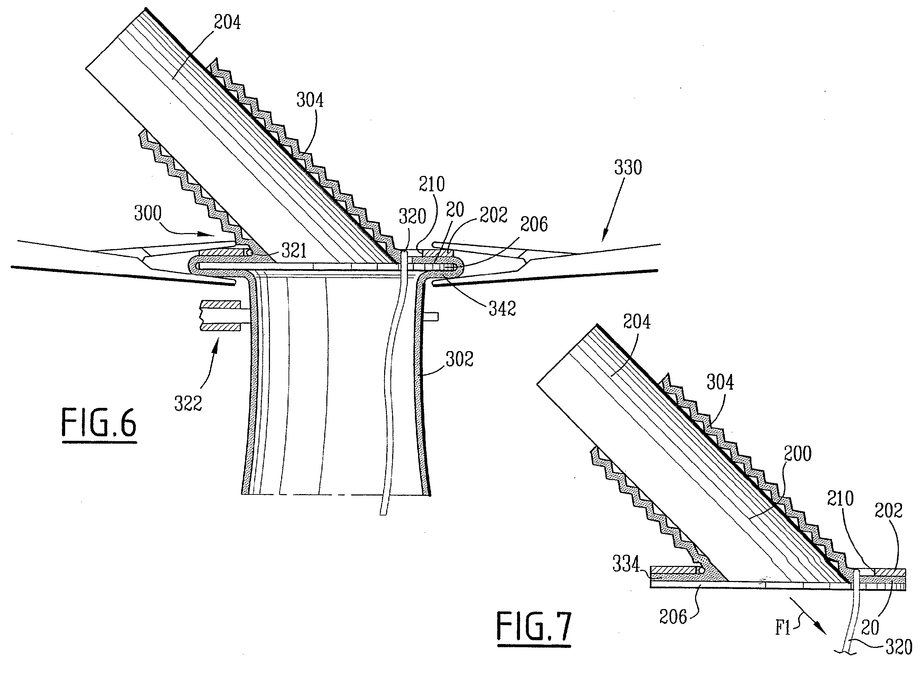

A l'étape suivante illustrée sur la figure 6, la rondelle 202 est engagée autour du bras 304 porté par la tige 204. Cette rondelle est amenée en appui sur la première couronne 20 reposant sur la surface supérieure du disque elliptique 206. Des pinces 330 sont mises en place pour assurer le plaque de la rondelle 202 sur la première couronne 20. A cet effet, l'un des mors de chaque pince s'applique sur la rondelle 202 alors que l'autre mors s'applique sur la partie de la jupe 302 reposant contre la surface inférieure du disque elliptique 206.

Alors que la jupe 302 est ainsi retenue de part et d'autre du disque 206, l'ébauche de prothèse est mise en forme par chauffage à la vapeur. L'ébauche de prothèse ainsi supportée par l'outil est soumise à un flux de vapeur et est portée à une température de 120°C pendant 20 mn. A l'issue de ce traitement, la forme de l'ébauche de prothèse imposée par l'outil la supportant se trouve marquée par l'action de la chaleur sur les fibres de polyester constituant le tricot de la prothèse.

La partie courante de la jupe 302 formant une surlongueur est ensuite sectionnée sensiblement suivant le pourtour du disque 206 grâce à des ciseaux ou un thermocuteur pour former un contour périphérique extérieur 334 essentiellement circulaire, comme illustré sur la figure 7.

Le corps d'outil 200 et la rondelle 202 sont alors dégagés de l'ébauche de prothèse.

A cet effet, le fil 320 est retiré et le corps d'outil 200 est dégagé suivant le sens de la flèche F1 sur la figure 7.

Un élément annulaire 424 est découpé dans une pièce d'étoffe tricotée avec un fil de même nature que celui utilisé pour le tronçon 118 et avec un point analogue. Il présente une forme extérieure légèrement elliptique.

L'élément annulaire 424 présente un contour extérieur identique au contour extérieur souhaité pour la collerette 19 de la prothèse. La section intérieure de l'élément annulaire 424 destiné à former la seconde couronne 24 est légèrement supérieure à la section elliptique de l'extrémité biseautée de l'élément tubulaire 304.

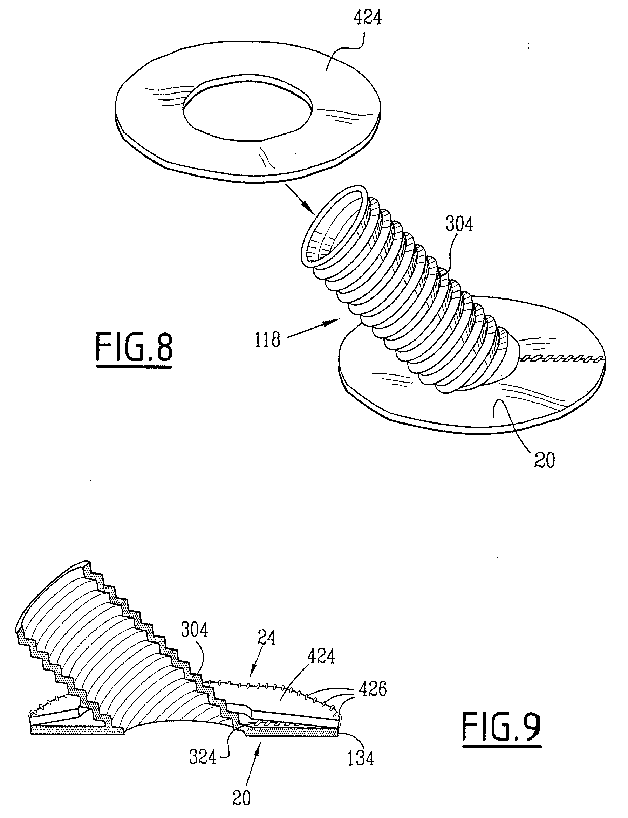

L'élément annulaire 424 est engagé autour de l'élément tubulaire 304 comme illustré sur la figure 8, jusqu'à être amené en contact avec la première couronne 20.

Comme illustré sur la figure 9, afin d'assurer l'assemblage de la prothèse, le contour extérieur 334 de la première couronne 20 est lié par une couture périphérique 426 à l'élément annulaire 424 afin de former la seconde couronne 24 de la prothèse. L'élément annulaire 424 est cousu sur la première couronne 20 alors qu'il est engagé autour du tronçon tubulaire 304 formant le corps 18.

La prothèse ainsi confectionnée est enduite de collagène en vue de son implantation dans un organisme vivant.

On conçoit que la prothèse ainsi réalisée présente une souplesse satisfaisante, et qu'elle est ainsi bien tolérée par l'organisme. De plus, sa réalisation par couture permet qu'elle soit réalisée pour un coût raisonnable.

Les prothèses décrites ici peuvent être réalisées à partir d'éléments textiles de toute nature adaptée, qu'ils soient tissés ou tricotés.

## Revendications

1. Prothèse à collerette (10), du type comportant un corps tubulaire (18) muni à une extrémité d'une collerette extérieure (19) adaptée pour l'anastomose de la prothèse sur un conduit tubulaire muni d'une ouverture, dans laquelle ladite collerette (19) est confectionnée dans un matériau textile et comporte au moins une couture (324, 426) assurant sa mise en forme à l'extrémité du corps tubulaire (18), ladite collerette comportant une première couronne (20) solidaire, suivant son contour intérieur, de l'extrémité du corps tubulaire (18) et une seconde couronne (24) entourant ledit corps tubulaire (18) et s'étendant sensiblement contre la première couronne (24) en étant reliées l'une à l'autre suivant leurs contours extérieurs, **caractérisée en ce que** la première couronne (20) est venue de matière avec le corps tubulaire (18), et les première et seconde couronnes (20, 24) sont liées entre elles par une couture périphérique extérieure (426).

2. Prothèse selon la revendication 1, **caractérisée en ce que** la première couronne (20) comporte au moins une couture transversale (324).

3. Prothèse selon la revendication 2, **caractérisée en ce que** ou chaque couture transversale (324) s'étend sensiblement radialement.

4. Prothèse selon la revendication 2 ou 3, **caractérisée en ce que** la première couronne (20) comporte une unique couture transversale (324).

5. Procédé de fabrication d'une prothèse à collerette (10) du type comportant un corps tubulaire (18) muni à une extrémité d'une collerette extérieure (19) adaptée pour l'anastomose de la prothèse sur un conduit tubulaire muni d'une ouverture, **caractérisé en ce qu'**il comporte les étapes suivantes :
- fabriquer une ébauche (300) de prothèse en matière textile comportant une jupe tubulaire (302) à une extrémité de laquelle débouche au moins un bras tubulaire (304) destiné à former le corps tubulaire (18) de la prothèse, le diamètre de la jupe tubulaire (302) étant supérieur au diamètre du bras (304),
- découper la jupe tubulaire (302) autour et à l'écart du bras tubulaire (304) pour former une première couronne (20) prolongeant le corps tubulaire (18),
- fabriquer dans un matériau textile un élément annulaire sensiblement plat (120), ledit élément ayant un contour extérieur sensiblement identique à celui de la première couronne ; et
- coudre ensemble ledit élément annulaire (120) à ladite première couronne par une couture périphérique (426) ménagée suivant leurs contours extérieurs.

6. Procédé selon la revendication 5, **caractérisé en ce qu'**il comporte pour la réalisation de la première couronne (20) une étape de mise en plis de la jupe tubulaire (302) autour d'un mannequin rigide (200) comportant au moins une partie (206) en forme de disque autour de laquelle est confectionnée la collerette (19).

7. Procédé selon la revendication 5 ou 6, **caractérisé en ce que** l'étape de fabrication de l'ébauche (300) comporte :
- une étape de fabrication d'une ébauche primaire (300) comportant une jupe tubulaire (302) à une extrémité de laquelle débouche un premier et un second bras tubulaires (304, 306), le premier bras (304) est destiné à former le corps tubulaire (18) de la prothèse ;
- une étape de découpe du second bras tubulaire (306) au niveau de la jupe tubulaire (302) ;
- une étape d'obturation par couture (324) de l'orifice obtenu par la découpe du second bras (306).
